# EUROPEAN PATENT APPLICATION

(11) **EP 1 396 713 A1**
(43) Date of publication of application: **10.03.2004**
(21) Application number: 02256216.9
(22) Date of filing: 09.09.2002
(51) Int. Cl.: G01N 1/00

(54) **Method and Apparatus for concentrating a gaseous substance**

(71) Applicant: Kore Technology Limited, Ely, Cambridge CB7 4EA (GB); Horiba, Ltd, Kyoto 601-8510 (JP)
(72) Inventor: Mullock, Stephen James, Impington, Cambridge CB4 9NU (GB); Matsumoto, Koichi, Kyoto (JP)
(74) Representative: Brunner, Michael John

(57) **Abstract**

A method of concentrating a gaseous substance, the method comprising the steps of: isolating a trap in which an analyte to be concentrated has been absorbed from a carrier gas passed through the trap; reducing the pressure within the trap to a first pressure desorbing the analyte from the trap; and diffusing the analyte released from the trap into an analyser operating at a second pressure lower than the first pressure.

## Description

The invention is primarily concerned with gas analysis, where low levels of compounds of interest are to be found in a matrix gas, usually air.

It is particularly relevant to the situation where the analyser is operated in at least a partial vacuum and the gas to be analysed is typically at atmospheric pressure. The exact pressures are not important, except that the invention relies upon a significant pressure difference between the analyser pressure and the pressure of the gas to be analysed. Typically, the analyser is a mass spectrometer which operates at pressures between 10⁻⁴ and 10⁻⁹ mbar.

It is often the case that the amount of gas that can be admitted to the analyser is limited by the matrix gas, but at the same time the more compound of interest that can be admitted, the greater the sensitivity of the measurement. In these cases, a device that increases the concentration of the compounds of interest within the matrix gas can increase the sensitivity of the measurement.

There are various ways to achieve this and two examples of known methods are described below. For clarity, in the discussion of the well known examples we assume that the compound of interest is benzene and the matrix gas is air. In other words, it is desired to measure low levels, perhaps a few parts per billion by volume, of benzene contamination in ambient air.

A first known example of gas concentration uses a trap and desorb concentrator. This is most often a concentrator at the front of a gas chromatograph. A small volume reservoir, to act as the trap, (typically of the order of 1 to 5 cc), is packed with a substance, usually a solid, which has the property that it absorbs and retains benzene very readily onto its surface, but does not retain the various air components, particularly oxygen, nitrogen, argon, carbon dioxide and water vapour. The trap material also has the property that the benzene will be released from the surface at some elevated temperature. The material is usually granular in order to maximise the surface area, but may take a variety of forms including a very thin layer of viscous fluid on a solid support. The design of such trap materials is a well established technology and they may be readily purchased in a form tailored for the analysis of various ranges of compounds in a variety of matrix gases.

A known volume of air is passed through the trap at room temperature, at a speed which allows the trap material to retain substantially all the benzene. Air that has passed through the trap is discarded. The sampled volume is much larger than the trap volume, typically a few litres. The air in the trap is now exchanged for an alternative gas, for example helium or hydrogen, that is suited to the analyser to be used. The trap is isolated by means of valves at each end and then heated to release the benzene. If the benzene has been trapped from say one litre of air and it is now released into a 5 cc volume of air or other gas, it can readily be seen that the concentration will be increased by the ratio of the sampled volume to trap volume, a factor of 200 in this example. The concentrated sample is then passed to the analyser, for example a gas chromatograph, by entraining in a carrier gas passing through the trap.

Typically, all of these processes take place at approximately the same pressure. The ability of the trap to concentrate is associated with firstly, a selective absorption and secondly, the ability to release the benzene again, typically by elevating the temperature. The temperature of the trap does not have to be room temperature, as, for example, a so called cryo-trap works without requiring a special chemistry for the absorbing surface. In this case, the trap temperature is so low that benzene condenses out of the air, but not so low that the air itself liquifies. The selectivity is by virtue of the different boiling points.

This trap and desorb technique is reasonably quantitive, as the concentrating power is directly related to an easy to measure volume ratio. It is also reasonably fast as the high surface area of the granular trap material removes benzene efficiently at a flow rate that allows the sampling to be achieved in a couple of minutes. However, it should be noted that, at very high volume ratios, there are problems with the trap material retaining all of the benzene from the sampled air. Thus, there are limits to the concentration that can occur and so the concentrating power cannot be arbitrarily high.

An alternative example uses a membrane concentrator. By a suitable choice of materials, it is possible to make a membrane which is selectively permeable. As an example, the permeability of silicone membrane material is about 100 times higher for benzene than for the major constituents of air. One strategy to create a concentrator with such a membrane is to create a pressure drop across it. Where the analyser of choice is a mass spectrometer, this is convenient as the ion source typically operates at low pressure in any case.

The membrane has an effective conductance that is approximately 100 times higher for benzene compared with air. The low pressure side of the membrane is pumped with a positive displacement or other non-selective pump so that any gas passing through the membrane is steadily removed. Assuming the pump rate is high compared with the effective conductance of the membrane for either component, the partial pressures on the downstream side are roughly proportional to the upstream pressure multiplied by the relevant conductance. Thus, although the partial pressures of both air and benzene are lower on the low pressure side of the membrane, the ratio of the benzene/air pressures is increased by about a factor of 100.

If the analyser pressure is low enough, it is possible to have two pressure steps and take advantage of two selective membranes, giving, a, potential enrichment of 10,000 times in our example for the benzene in air. A realistic example would involve a first membrane separating atmospheric air from an intermediate vacuum of about 1 mbar, with a second membrane separating this region from the mass spectrometer source operating below 10⁻⁴ mbar.

The membrane based inlet has various advantages: the sampling is quasi-continuous and no valve structures are required. However, the membrane response time can be rather slow for some species, although the trap can potentially provide a better concentration ratio and also has the possibility of using a temperature programmed desorption to give additional sample information. There is no reason why both the above methods should not be employed in series; a trap and desorb followed by a membrane concentrator.

In a known double membrane concentrating inlet designed for use with a portable mass spectrometer of limited pumping capacity, sample air at atmospheric pressure is drawn past a first membrane, usually of silicone polymer. A second, inner membrane separates the intermediate vacuum from the mass spectrometer. There is an ultra high vacuum (UHV) valve between the two membranes mounted as close as possible to the inner membrane. When shut this allows the intermediate vacuum space to be vented to air without overloading the spectrometer pumps. This means that the miniature intermediate vacuum pump, which is mechanical and therefore subject to wear, only needs to operate during a measurement. Also the outer membrane may be easily changed without venting the mass spectrometer. By contrast the inner membrane cannot be changed without venting the mass spectrometer.

The mass spectrometer is designed to be sealed between major services, which avoids the need for heavy power hungry pumps that are only very rarely used. It is only ever vented and pumped again upon return to the factory. This greatly simplifies management of the vacuum for the user as well as saving cost, but the implication is that the inner membrane cannot be changed in normal use.

The presence of intermediate vacuum region, as well as allowing the option of two membrane concentration stages, is convenient for dealing with the practical difficulty of leaking such a small quantity of sample across some ten orders or magnitude of pressure ratio (1 bar to 1-5 x 10⁻⁷ mbar). It is a great deal easier to engineer the correct conductance of the inner pressure step working from 1 mbar rather than 1 bar. This point applies both when the pressure step is across a membrane and in the case when a simple flow restrictor, such as an aperture, is used.

The double membrane arrangement is very effective in many situations, but there are certain drawbacks. There are not many membrane materials that work well over a wide range of analytes. The one that most workers return to is silicone, which is fine for many volatile organic compounds but not so good for polar compounds such as the alcohols. Also silicone is relatively permeable to water vapour so that excessively humid samples must be dried before being presented to the inlet to avoid overloading the spectrometer vacuum pump. There is also no simple way to increase the sensitivity further; a third membrane would require another intermediate vacuum region held at around 10⁻¹³ to 10⁻⁴ mbar which is hard to achieve with a portable pump. Some compounds of potential interest diffuse very slowly in the membrane material leading to an unacceptably slow time response. Part of the problem is that the membrane materials have to engineered to adjust two properties simultaneously, the affinity to the compounds of interest (formally expressed as a partition coefficient) and the diffusivity.

An obvious extension to increase sensitivity is to add conventional trap and desorb concentrator to the beginning of the double membrane concentrator. Several problems can be eased this way, at the expense of extra complexity. The total concentrating power may be increased and the water vapour may be flushed out of the trap prior to the desorb phase, protecting the spectrometer. However, the speed is still slow and with two membranes there is still a very strong bias against polar compounds. The situation would be greatly improved if the outer membrane could be dispensed with altogether, but then there is the question of whether enough total concentrating power remains.

A yet further example is disclosed in US-A-5142143 which discloses a preconcentrator for analysing trace constituents in gases where a sample gas is introduced to a confined sorbent which thereafter is evacuated by a vacuum pump, and a low pressure carrier gas passes through the sorbent while it is desorbing, wherein the desorb trace constituents are carried by the carrier gas to a detector that operates at low pressure, such as a mass spectrometer. In particular, US-A-5142143 requires that a carrier gas, moving at a predetermined gaseous mass flow rate which is substantially less than the gaseous mass flow rate of the sample gas, is used to carry the desorbed trace constituents into the detector. This requires an additional low pressure gas supply to be provided, thereby increasing the complexity and size of the arrangement.

It is an aim of the present invention to provide a method and apparatus for concentrating a gaseous substance and which overcomes the problems associated with the arrangements described above.

According to the present invention, there is provided a method of concentrating a gaseous substance, the method comprising the steps of:
isolating a trap in which an analyte to be concentrated has been absorbed from a carrier gas passed through the trap;
reducing the pressure within the trap to a first pressure
desorbing the analyte from the trap; and
diffusing the analyte released from the trap into an analyser operating at a second pressure lower than the first pressure.

Thus, the present invention concerns a variation to the trap and desorb method and is particularly suited to use with a mass spectrometer or other vacuum analyser. The trap retains the compound of interest, i.e. the analyte, and the reduction in pressure removes only the carrier gas, such that the concentrating power of the trap is greatly enhanced. This is best achieved by evacuating the trap to a pressure between the sampling pressure and the analyser pressure, so that during the analysis phase, the intermediate pressure region can act as a reservoir of preconcentrated gas for the analyser. The main advantage of the present invention is that a more sensitive measurement can be made with a small quantity of the compound of interest and this can be accomplished faster than using conventional methods. A further advantage is that an additional source of carrier gas is not required, thereby reducing the size of the apparatus which is important in a portable device.

The method may further comprise the step of passing the carrier gas and the analyte through the trap and, more preferably, the pressure of the carrier gas and analyte is at a third pressure which is greater than the first pressure.

The carrier gas and analyte may be passed through a selectively permeable membrane prior to passing them into the trap. Alternatively or additionally, the analyte may be passed through a selectively permeable membrane after the desorb step.

The method may also comprise the step of flushing the trap with a dry gas prior to reducing the pressure in the trap.

It is preferable that the desorption of the analyte is effected by raising the temperature of the trap.

The present invention also provides a device for concentrating and analysing a carrier gas, the device comprising:
a trap in which an analyte can be retained;
means for desorbing the analyte from the trap;
a body into which the analyte passes from the trap;
a vacuum pump for reducing the pressure in the trap and/or in the body; and
an analyser into which the concentrated analyte is passed for analysis.

Preferably the means for desorbing the analyte is a heater.

The trap may include a valve means at its inlet, through which the carrier gas and analyte are passed.

The analyser may be a mass spectrometer.

The device may also comprise an analyser valve between the body and the analyser and may include a pump valve between the body and the vacuum pump.

The device may further comprising a flow restrictor between the body and the analyser. The flow restrictor is preferably a selectively permeable membrane.

By relying on diffusion to transport an analyte from the trap to the mass spectrometer inlet, no additional carrier gas need be provided. The coefficient of diffusion is approximately proportional to pressure. If the pressure is high enough for a carrier gas to transport the analyte, it follows that the diffusion will be slow. However, in the present invention, the trap is sited close to the inlet and the pressure is made low enough that the analyte reaches the mass spectrometer inlet quickly. At the same time, providing that the total volume that the analyte diffuses into is kept small, the greatly reduced quantity of matrix gas present when the trap is desorbed leads to a much higher concentrating power.

A good example of a case where the invention can be usefully employed is the case of a portable mass spectrometer used for analysis of trace gases in air. For the mass spectrometer to be portable, the pumping requirements must be minimised. An effective way to do this is to operate the mass spectrometer at a good vacuum, say 1 to 5 x 10⁻⁷ mbar, and pump using a small ion pump. In this case, the power requirements of the pump are directly related to the rate at which a sample is admitted. Therefore, only a very small quantity of sample gas is admitted, typically around 5 x 10⁻⁷ mbar litre/second. The bulk of gas admitted is matrix gas, but the sensitivity of the measurement is proportional to the partial pressure of volatile organic in the spectrometer. Therefore this is a good example of a case where a concentrating inlet is required to reach useful detection limits, where useful in this context is often measured in a few parts per billion (ppb).

One example of the present invention will now be described with reference to the accompanying drawings in which:
Figure 1 is a schematic representation of an example of a prior art concentrator;
Figure 2 is a schematic representation of a second example of a prior art concentrator;
Figure 3 is a schematic representation of a third example of a prior art concentrator;
Figure 4 is a schematic representation of the present invention;
Figure 5 is a schematic representation of a further example of the present invention; and
Figure 6 is a schematic cross-sectional view through an example of the present invention;

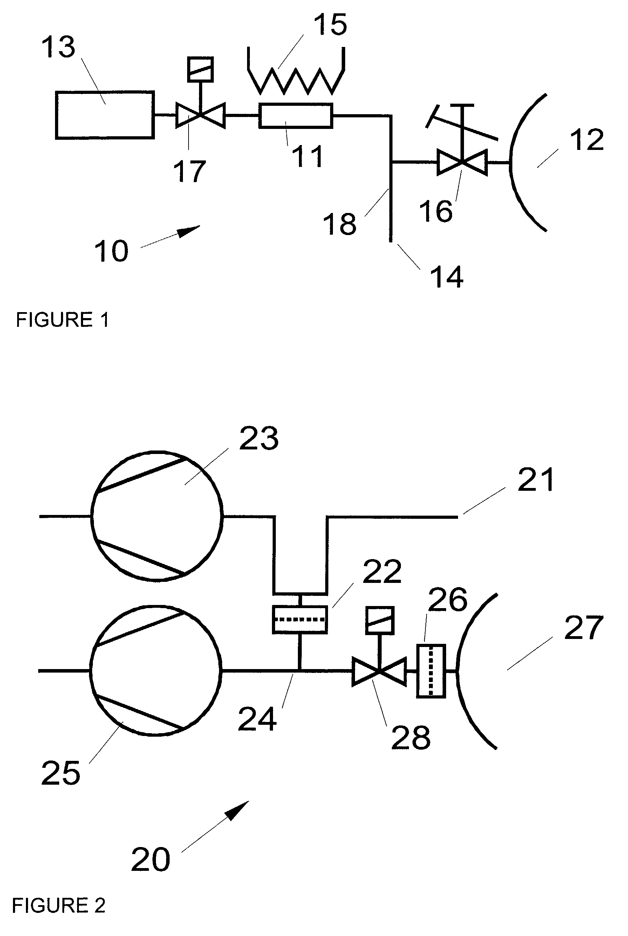

Figure 1 shows a simple prior art system 10 to transfer sample from a trap 11 in which the sample has already been retained into a mass spectrometer 12. The system is also provided with a gas supply 13, an exhaust 14, a heater 15 and valves 16, 17. Valve 16 is a leak valve that admits a small portion of the flow into the mass spectrometer (limited by the capacity of the high vacuum pumps required for the mass spectrometer). Heater 15 heats the trap 11 to release the analyte from the absorbent within the trap, valve 17 is opened to allow transport gas from the gas supply 13 to be admitted to the trap 11. This pushes the analyte out of the trap 11, as a relatively concentrated "plug" of gas, and transports it to the mass spectrometer inlet valve 16. Region 18 is normally at, or a little above, atmospheric pressure during this process. Fine control of the timing of opening of valve V2 allows the sample plug to be stopped at the inlet valve 16, if time is needed for the analysis. There are also possible variations that use a pump instead of excess pressure in the gas supply to drive the transport gas. It should be noted that the concept of a transport gas only works if the diffusion of the analyte is relatively slow compared with the gas velocity. At the opposite extreme, if the diffusion rate is very fast, the analyte would behave essentially independently of any other gas in the system and there would be no sample plug.

US 5,142,143 uses a variation of the method described in Figure 1. This document teaches explicitly the use of a transport gas at a reduced pressure, having reduced the trap pressure prior to desorption, in an attempt to further increase the concentrating power.

Figure 2 shows a typical double membrane concentrator 20, as implemented by the Applicant in its existing portable battery powered mass spectrometer. The sample to be analysed, usually air at or near atmospheric pressure, is drawn from an inlet 21 past a first membrane 22 by sampling pump 23. Some sample gas permeates through the membrane 23 into region 24 which is maintained at a lower pressure, typically 2 mbar, by pump 25. The membrane is selected so that its conductance is higher for the compounds of interest than for the matrix gas and therefore the relative concentration of compounds of interest to matrix gas is increased. A further concentrating step occurs as gas from the intermediate pressure region 24, permeates through the second membrane 26 into the mass spectrometer 27. A valve 28 is held open during this sampling process. Its presence is necessary to protect the spectrometer 27 from excessive gas load if the pressure in region 24 rises. This happens when the spectrometer is not in use because pump 25 is normally switched off when not needed, both to save battery power and to reduced wear. Also when valve 28 is shut, the outer membrane 22 may be exchanged without compromising the spectrometer vacuum.

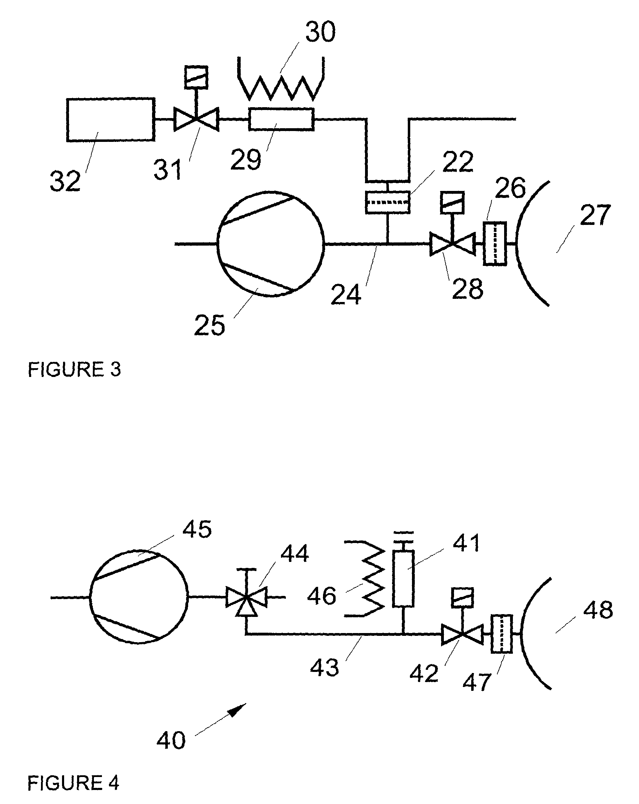

An alternative arrangement would be to add a trap 29, a heater 30, a valve 31 and a gas supply 32 to the existing double membrane concentrator in a conventional way, in which case the arrangement would end up as shown in Figure 3. This is functionally similar to Figure 1, except the leak valve has been replaced by the double membrane concentrator.

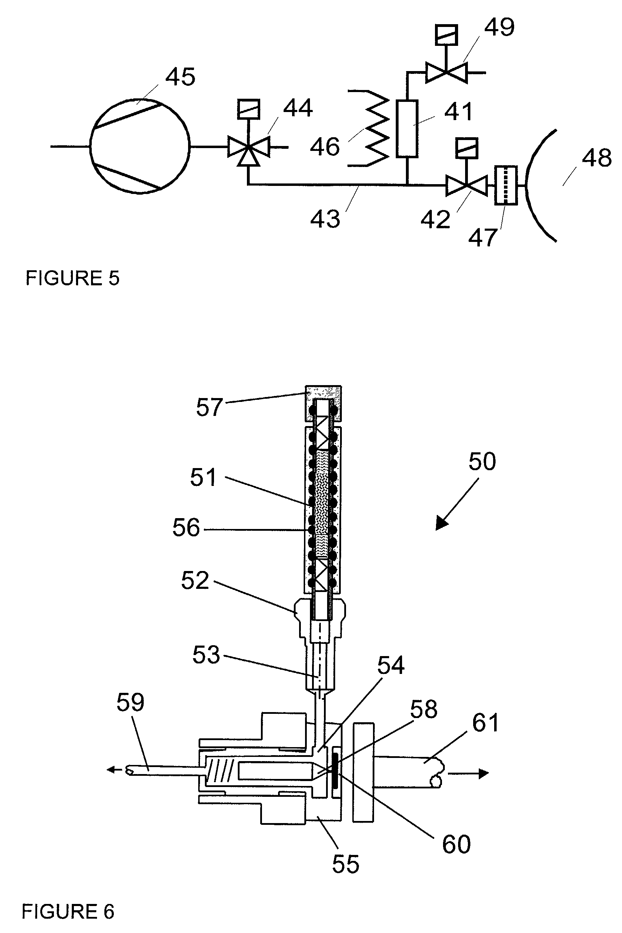

Figure 4 is a schematic of a simple implementation of the invention. In this example, a gas concentrator 40 is connected to a trap 41 that has already been loaded with sample. In a practical implementation, this is by a quick-connect fitting (see Figure 6). It is envisaged, however, that the trap could form an integral part of the device such that the gas to be sampled is passed through the trap, whilst it is connected to the remainder of the device of the present invention. To begin with a mass spectrometer inlet valve 42 is shut and region 43 is at atmospheric pressure as a result of just having fitted the trap. A valve 44 is opened to connect region 43 to a vacuum pump 45 and the pressure is reduced to, typically, 0.5 to 0.1 mbar. This is the base pressure of the small battery powered pump that the Applicant's currently employ.

The valve 44 is then changed so that the region 43 is sealed off. The valve 42 is opened and the trap 41 is heated with a heater 46. Analyte is released from the trap and diffuses throughout the region 43. This diffusion process is relatively fast because of the low pressure. The gas in region 43 can then permeate through a membrane 47 and into the mass spectrometer 48. A series of one or more mass spectra can then be taken.

There is a delay between the start of the heating and the maximum signal followed by a slow drop off as the gas in the region 43 is slowly pumped into the mass spectrometer. Several factors affect the delay, the time taken for the absorbent in the trap to heat, diffusion from the trap to the membrane and time to diffuse through the membrane. When the data has been collected, the valve 42 may be shut and the valve 44 opened to vent region 43. The trap 41 can now be replaced with a fresh one, for further analysis to take place.

The design used to implement this schematic seeks to minimise the distance between the trap and the inner membrane 47 so that analysis time is as short as possible. The better the vacuum in the region 43 before the heating starts, the higher the concentrating ratio and the faster the diffusion will be. The limit will be determined by the size of pump used and the room temperature out-gassing rate. Once the pressure is so low that the mean free path of gas molecules is longer than the tube diameter, there will be diminishing returns in the sense that further pressure reduction will not speed up the diffusion process much further.

Note that in contrast to the prior art, there is no sample "plug" to be pushed or entrained into the mass spectrometer. The concentrating power of the trap depends on the ratio of the sampled volume when loading the trap to the whole volume of region 43, as well as on the pressure ratio. Thus it is desirable to minimise the volume of the region 43.

The inner membrane 47 experiences very little mechanical load because of the low pressure difference in this setup. This means that there is the possibility to use much thinner material which will speed up the process of diffusion through the membrane. There may be more choice to the membrane in other ways too if mechanical strength is less of an issue.

There are of course many variations that could be envisaged. Example 1: The plumbing to load the trap could be included. Example 2: multiple traps could be processed at once to speed the overall cycle; one trap would be pumping, another being analysed and a third in the sample phase and so on. Also the membrane 47 could be replaced by an alternative flow restrictor.

An arrangement that would include the sampling is depicted in Figure 5. The top of the trap is now sealed with a valve 49, which, when open, would allow the vacuum pump to draw air through the trap to load it.

Whilst Figures 4 and 5 show a theoretical arrangement, an actual example is shown in Figure 6. The device 50 includes a trap 51 which is connected, by means of a quick release seal 52, to a passageway 53 which is in communication with chamber 54 in body 55. The trap 57 is surrounded by a heater 56 which can be operated to raise the temperature of the trap, for example, to initiate desorption of the trapped material. The upper end, in Figure 5, of the trap is sealed by means of end seal 57. In this example, the carrier gas and analyte have already been passed through the trap, which has then been connected to the body 55 for analysis.

Main body 55 is provided with an ultra high vacuum isolation valve 58 which seals chamber 54 from a membrane 50. The chamber 54 is also connected, via passage 59 to a vacuum pump (not shown). The inner membrane 60, which may be selectively or non-selectively permeable, is located between valve 58 and a passageway 61 which leads to, in this example, a mass spectrometer. The mass spectrometer is operated at a lower pressure to that found in chamber 54, as described with regard to Figure 4, thereby enabling diffusion of the analyte from trap 51 into the spectrometer for analysis.

The present invention provides a solution to the problems associated with the double membrane concentrator and an improvement to conventional trap and desorb concentrators. The first membrane is removed and a conventionally engineered trap put in its place. The sample, at atmospheric pressure, is loaded onto the room temperature trap with the UHV valve protecting the inner membrane and spectrometer. After sampling, the trap can be flushed with dry gas if necessary to reduce water vapour. Then the intermediate vacuum space, which now includes the trap, is evacuated with the existing miniature pump from 1 bar to less than 1mbar, increasing the potential concentrating power of the trap by a factor of 1000 over and above the volume ratio of sampled air to trap volume. The pump is sealed off and the trap is heated to release the compounds of interest. There is no carrier gas to push the analyte towards the inner membrane, however, at these reduced pressures, diffusion rates are much higher, owing to the longer mean free path, therefore the gas at the inner membrane rapidly reaches an equilibrium where the partial pressures of the compounds of interest are proportional to the absolute quantities that were absorbed onto the trap. It should be noted that this implies that no special measures are necessary to achieve a precise pressure during the evacuation phase and that the conductance from the intermediate pressure region, including the trap, into the mass spectrometer is still very small compared with the trap volume, so the partial pressures of analyte remain relatively stable whilst an equilibrium is established across the inner membrane and during the measurement.

The concentrating power of the present invention can easily be made much greater than that of a single membrane. Therefore, in this example, the overall sensitivity of the instrument is increased by over an order of magnitude even though one of the concentrating membranes has been removed and even for compounds which work well with the membrane. For polar compounds, the gains may be a great deal higher again. In many cases, it may not even be necessary to have an inner membrane; a flow restrictor with the correct conductance could be used instead.

The trap material is more easily engineered for a wide range of applications as the diffusivity is not a factor, in contrast to membrane requirements. The desorb phase may be a simple step change in temperature, designed to desorb all compounds of interest, or it may be some controlled time profile such as ramp. This can be used to achieve a greater degree of discrimination between compounds which might have similar mass spectra but different temperature dependence in the partition coefficient.

Automation of the trap, evacuate, desorb process could be achieved simply in a variety of ways. If the positive displacement pump has a well defined pumping speed, there is the possibility of using it both for sampling and for the evacuation. In this case, a single electrically operated valve in place of the end seal might be all that is required for a simple cycle that does not require a dry flush. A variety of valves and other equipment developed for gas chromatography could easily be adapted for use in more complex schemes.

An additional advantage of a trap based sampling system in that the traps may be loaded with very simple apparatus quite separately from the analysis. Even when using a portable gas analyser on-site, it may be convenient to load a set of traps at locations around a site without having to carry the mass spectrometer to each point and then bring the traps back to a central point, perhaps a vehicle, for analysis. This mode of operation is compatible with the present invention.

Although the discussion has concerned gas analysis, the gas in question may be head space or sparging gas that has been in contact with liquids or even solid material. Therefore it can be seen that the invention might be employed, for example, for water or soil analysis. Some trap materials may even be suitable for exposure directly to fluids.

The combination of remote loading of the traps, together with the ability to deal with humid samples at very high sensitivity may make this a particularly suitable concentrator for use in breath analysis. Sampling could be achieved by breathing a known volume directly through the trap.

Of course the present invention may be used with any analyser that operates with a suitable vacuum and may, in particular, be used with a mass spectrometer that employs further pressure steps for other purposes, for example a differentially pumped ion source.

## Claims

1. A method of concentrating a gaseous substance, the method comprising the steps of:
isolating a trap in which an analyte to be concentrated has been absorbed from a carrier gas passed through the trap;
reducing the pressure within the trap to a first pressure
desorbing the analyte from the trap; and
diffusing the analyte released from the trap into an analyser operating at a second pressure lower than the first pressure.

2. A method according to claim 1, further comprising the step of passing the carrier gas and the analyte through the trap.

3. A method according to claim 2, wherein the carrier gas and the analyte are at a third pressure which is greater than the first pressure.

4. A method according to any one of the preceding claims, further comprising the step of passing the carrier gas and the analyte through a selectively permeable membrane prior to passing them into the trap.

5. A method according to any one of the preceding claims, further comprising the step of passing the analyte through a selectively permeable membrane after the desorbing step.

6. A method according to any one of claims 1 to 4, further comprising the step of passing the analyte through a non-selective flow restrictor after the desorb step.

7. A method according to any one of the preceding claims, further comprising the step of flushing the trap with a dry gas prior to reducing the pressure in the trap.

8. A method according to any one of the preceding claims, wherein the desorption of the analyte is effected by raising the temperature of the trap.

9. A device for concentrating and analysing a carrier gas, the device comprising:
a trap in which an analyte can be retained;
means for desorbing the analyte from the trap;
a body into which the analyte passes from the trap;
a vacuum pump for reducing the pressure in the trap and/or in the body; and
an analyser into which the concentrated analyte is passed for analysis.

10. A device according to claim 9, wherein the means for desorbing the analyte is a heater.

11. A device according to either claim 9 or claim 10, wherein the trap includes a valve means at its inlet, through which the carrier gas and analyte are passed.

12. A device according to any one of the preceding claims, wherein the analyser is a mass spectrometer.

13. A device according to any one of claims 9 to 12, further comprising an analyser valve between the body and the analyser.

14. A device according to any one of claims 9 to 13, further comprising a pump valve between the body and the vacuum pump.

15. A device according to any one of claims 9 to 14, further comprising a flow restrictor between the body and the analyser.

16. A device according to claim 15, wherein the flow restrictor is a selectively permeable membrane.
